# EUROPEAN PATENT SPECIFICATION

(11) **EP 1 287 808 B1**
(45) Date of publication and mention of the grant of the patent: **26.07.2006**
(21) Application number: 02405730.9
(22) Date of filing: 28.08.2002
(51) Int. Cl.: A61K 8/06, A61K 8/58, A61K 8/67, A61K 8/898, A61K 8/90, A61Q 5/00, A61Q 19/00

(54) **Stable aqueous emulsions of alkoxytrimethylsilane fluids**
Stabile wässrige Emulsion von Alkoxytrimethylsilane-Fluids
Emulsion aqueuse stable des fluides alkoxytriméthylsilane

(30) Priority: 30.08.2001 US 942483
(43) Date of publication of application: 05.03.2003
(73) Proprietor: Clariant Life Science Molecules (Florida) Inc., Gainesville, FL 32609 (US)
(72) Inventor: Legrow, Gary E., Newberry, Florida 32669 (US)
(74) Representative: Kachholz, Traudel

(56) References cited:
- EP-A- 0 576 748
- WO-A-94/14404
- US-A- 5 847 179

## Description

### FIELD OF THE INVENTION

The present invention relates to aqueous emulsions comprising an alkoxytrimethylsilane that can be used for delivering alkoxytrimethylsilanes to surfaces on the human body, including, but not limited to, the stratum corneum and the hair. More particularly, this invention relates to aqueous emulsions comprising an alkoxytrimethylsilane that remains intact with time under normal ambient conditions with no measurable change in the discontinuous phase, no particle aggregation, and no chemical transformations.

### BACKGROUND OF THE INVENTION

A wide range of long chain aliphatic alcohols are used in cosmetic formulations, including skin care and hair care products, because of some of the inherently desirable characteristics of these materials including occlusivity, substantivity, water repellency, and ultraviolet radiation protection. Long chain aliphatic alcohols, possessing these characteristics, typically have carbon chain lengths ranging from 2 upwards to more than 20 carbon atoms. When pure, all of these alcohols are solids at room temperature. In formulating cosmetic products, higher melting ingredients are frequently melted, such as in a hot room, and then formulated while in the molten state. The requirement of performing this melting step represents a significant disadvantage to the use of solid ingredients in formulating cosmetic products.

It therefore would represent a significant advance in the state of the art if a composition that is useful in providing the beneficial characteristics of solid long chain aliphatic alcohols could be developed that does not also possess the drawback of requiring melting prior to formulation. In this regard, mention is made here of United States Patent No. 5,847,179, which discloses liquid alkoxytrimethylsilanes, trimethylsilyl derivatives of solid long chain aliphatic alcohols, that can be formulated readily into cosmetic products at room temperature. An even greater advance in the state of the art of cosmetic formulations, however, would be the ability to formulate these liquid long chain aliphatic alkoxytrimethylsilanes under ambient conditions into aqueous emulsions and retain the liquid alkoxytrimethylsilane structure intact.

Mention is also made of Fisher, United States Patent No. 6,074,470 which teaches a non-silicone emulsifying system consisting essentially of a non-ionic primary surfactant having an HLB greater than 13 and a secondary non-ionic co-surfactant having an HLB less than 11 for emulsifying alkoxysilanes useful as water repellents.

### SUMMARY OF THE INVENTION

The present invention meets the problems of the prior art and provides stable aqueous emulsions comprising fluid alkoxytrimethylsilanes in the discontinuous oily phase and a method for their preparation. The aqueous emulsions comprise an alkoxytrimethylsilane, an organosilicone hydrophobic emulsifier, an organosilicone hydrophilic emulsifier, and water. The aqueous emulsions exhibit improved stability in that the discontinuous particles do not increase in size with age, over at least a period of three months, and there is no apparent indication of hydrolysis of the alkoxytrimethylsilane.

### DETAILED DESCRIPTION OF THE INVENTION

The aqueous emulsions of the present invention are stable and do not phase separate. They have constant particle size over at least a three-month period under ambient conditions. In the case of fluid alkoxytrimethylsilanes derived from solid alcohols, another measure of stability of the aqueous emulsion is the absence of solid alcohol over time, the presence of which would be accounted for by hydrolysis of the alkoxytrimethylsilane.

The present invention provides an aqueous liquid alkoxytrimethylsilane containing emulsion comprising:
(a) a continuous phase comprising water;
(b) a discontinuous phase comprising at least one liquid alkoxytrimethylsilane, wherein the discontinuous phase forms particles which are uniformly dispersed in the continuous phase; and
(c) a dual emulsifier system consisting essentially of an organosilicone hydrophobic surfactant and an organosilicone hydrophilic surfactant.

The organosilicone hydrophobic emulsifying agent is believed, although the inventor does not wish to be bound to any theory, to prevent diffusion of the liquid organic and/or alkoxytrimethylsilane particle into the continuous aqueous phase. The organosilicone hydrophilic emulsifying agent is believed, although again the inventor does not wish to be bound to any theory, to prevent coalescence of the discontinuous organic and/or alkoxytrimethylsilane particles.

The alcohol, from which the liquid alkoxytrimethylsilane is formed, has an aliphatic hydrocarbon structure with from twelve to more than 22 carbon atoms which may be acyclic, cyclic or both, and which may contain unsaturated groups.

The amount of liquid alkoxytrimethylsilane in the discontinuous phase of the aqueous emulsion may range from 1 to 100 percent. In addition to the alkoxytrimethylsilane, the present invention contemplates the inclusion of inert organic diluents such as, for example but not limited to, soybean oil, mineral oil or other vegetable oils, or organic esters such as isopropylmyristate, and the like, or mixtures thereof, in the discontinuous phase of the emulsion. The amount of the discontinuous phase in the aqueous emulsion may range from about 5 to about 50 percent.

Liquid alkoxytrimethylsilanes, useful in the aqueous emulsions of the present invention and in cosmetic applications, include, but are not limited to, those containing alkoxy groups with aliphatic hydrocarbon structures with from 12 to more than 22 carbon atoms, which may be cyclic, acyclic or both, and which may contain unsaturated groups. Suitable examples include retinoxytrimethylsilane, stearoxytrimcthylsitanc, cetoxytrimethylsilane, cctearoxytrimethylsilane; arachidoxytrimethylsilane, behneoxytrimethylsilane, palmitoleooxytrimethylsilane, oleoxytrimethylsilane, linoleoxytrimethylsilane, linolenoxytrimethylsilane, arachidonoxytrimethylsilane, erucoxytrimethylsilane and mixtures of any of the foregoing. Especially suitable examples are retinoxytrimethylsilane or stearoxytrimethylsilane. In preferred embodiments of the present invention, the alkoxytrimethylsilane fluids can be prepared according to the methods disclosed in LeGrow et al., United States Patent No. 5,847,179.

Organosilicone hydrophobic surfactants suitable for use with the aqueous emulsions of the present invention are of the alkyl-silicone-polyoxyalkylene type. Alkyl-silicone-polyoxyalkylene copolymers are particularly suited for encapsulation of hydrocarbons and/or silanes in aqueous emulsions because of their affinity for both hydrocarbon and silane compositions. The concentration of the organosilicone hydrophobic surfactant in the emulsion should be sufficient to continuously cover the surface of the discontinuous particles, typically one to three molecules per 100 square Angstroms of surface area.

Preferred are aqueous emulsions wherein the organosilicone hydrophobic surfactant is of an alkyl PEG/PPG-x/y dimethicone copolymer or an alkyl PEG/PPG-x/y methicone polymer, or an alkyl bis(PEG/PPG-x/y) dimethicone polymer, wherein alkyl is a saturated hydrocarbon substituent with from 6 to 18 carbons, PEG is polyoxyethylene, PPG is polyoxypropylene, and x and y may independently range from to 20, and wherein said organosilicone hydrophobic surfactant is present in the emulsion at a concentration ranging from one to three molecules per 100 square Angstroms of surface area of the alkoxytrimethylsilane particles.

Also very good properties show aqueous emulsions wherein the organosilicone hydrophilic surfactant comprises a water soluble trisiloxane PEG-x copolymer, wherein the trisiloxane is (Me₃SiO)ZMeSi-, Me is methyl, PEG is polyoxyethylene, and x may range from 1 to 20, and wherein said organosilicone hydrophilic surfactant is present in the emulsion at a concentration of from two to six molecules per 100 square Angstroms of surface area of the organosilicone hydrophobic surfactant treated particles.

Especially preferred are aqueous emulsions wherein the discontinuous phase is substantially comprised of particles having a diameter of less than about 500 nanometers.

Organosilicone hydrophobic surfactants of the alkyl-silicone-polyoxyalkylene type, which are suitable for use in the practice of the present invention and which are commercially available include, but are not limited to, DC-5200 which is Lauryl PEG/PPG-18/18 methicone copolymer, produced by Dow Coming Corporation; Abil® EM-90 which is Cetyl PEG/PPG-1/1 dimethicone copolymer, produced by Goldschmidt AG and SilCareTM 140M30 Caprylyl Bis-(PEG/PPG-20/20) dimethicone copolymer, produced by Clariant. This latter product is an example of a class of surfactants disclosed in currently pending, commonly assigned, United States Patent Application Serial No. 09/549,325 filed an November 13, 2000 (attorney docket 577-124) by the same inventor.

Organosilicone hydrophilic surfactants suitable in these emulsions are of the water-soluble silicone-polyoxyethylene type, of the general formula:

(Me₃SiO)₂MeSi(CH₂)₃O(CH₂CH₂O)ₓMe

wherein x may range from 6 to about 20. Water-soluble silicone-polyoxyethylene copolymers are particularly suited to prevent coalescence of the discontinuous organic and/or alkoxytrimethylsilane particles because of their affinity for the alkoxytrimethylsilane and their ability to reduce the surface tension of water in their environment. The concentration of the hydrophilic surfactant in the emulsions of the present invention should be sufficient to cover the surface of the hydrophobic surfactant treated discontinuous particles, typically two to six molecules per 100 square Angstroms of surface area. An organosilicone hydrophilic surfactant, which is suitable for use with the aqueous emulsions of the present invention, and which is commercially available is, but is not limited to, Silt,are 1-M 140M40 Trisiloxane PEG-10 copolymer produced by Clariant. Reference is also made to NII, Randall M., ed., "Silicone Surfactants," Marcel Dekker Surfactant Science Series, vol. 86, 1999, pp. 241-48 which although related to herbicide formulations provides a further description of these materials and commercially available examples that are useful in the practice of the present invention.

The aqueous emulsions may further comprise one or more of the following components:
(d) a biocide;
(e) a buffering agent;
(f) a fragrance;
(g) a colorant;
(h) a foaming agent;
(i) an anti-foaming agent; and/or
(j) a thickener.

The aqueous emulsions of the present invention may be used in cosmetic formulations for delivery of the liquid alkoxytrimethylsilane, which they contain, to the surface of the skin or hair. The spreadability and penetrating characteristics of liquid alkoxytrimethylsilanes, as is well known to those skilled in the art, then provide more efficient delivery to the skin or hair, thereby making these emulsions more efficacious.

Once in the upper layers of the stratum corneum or on the hair, these emulsions break liberating liquid alkoxytrimethylsilanes, which are then hydrolysed by contact with water liberating the parent solid alcohols.

### DESCRIPTION OF THE PREFERRED EMBODIMENTS

The following examples are presented to illustrate the present invention. These examples are not to be construed to limit the scope of the appended claims in any manner whatsoever.

### COMPARATIVE EXAMPLE 1

An aqueous emulsion of liquid stearoxytrimethylsilane was prepared by the following method. Forty grams of stearoxytrimethylsilane were mixed with 1.4 grams (0.117 mmole) of SilCareTM Caprylyl Bis(PEG/PPG-20/20) dimethicone copolymer forming a hazy solution (1). Over a period of 10 minutes, solution (1) was slowly added to twelve grams of water using a Tissue-Tearor homogenizer running in the range of 4000-8000 rpm. After complete addition, the homogenizer was run for an additional 20 minutes. To this mixture was added 46.6 grams of de-ionized water, using the homogenizer, over a period of 10 minutes. After complete addition, the homogenizer was run for an additional 20 minutes. Finally, 0.1 gram of sodium bicarbonate was added to the mixture and blended with the homogenizer for 10 minutes.

A sample of the above aqueous emulsion was examined with a Fisher optical microscope using 1000x magnification (1 unit an the scale = 1 micrometer). The discontinuous "oil-phase" particles were observed to be of varying size, ranging from about 200 to about 500 nanometers in diameter. Within 12 hours, this sample showed a large phase separation and solid crystalline stearyl alcohol started to form. Within 24 hours the Sample thickened significantly.

### EXAMPLE 1

An aqueous emulsion of liquid stearoxytrimethylsilane was prepared by the following method. Twelve (12) grams of water were mixed with 0.5 grams (0.665 mmole) of SilCareTM 140M40 Trisiloxane PEG-10 copolymer forming a clear solution (1). Forty grams of stearoxytrimethylsilane were mixed with 2.8 (0.272 mmole) of SilCareTM 14M30 Caprylyl Bis(PEG/PPG-20/20) dimethicone copolymer until a hazy mixture was formed (11). Over a period of 10 minutes, mixture (11) was slowly added to solution (1) using a Tissue-Tearor homogenizer running in the range of 4000-8000 rpm. After complete addition, the homogenizer was run for an additional 20 minutes. To this mixture was added 44.7 grams of deionized water, using the homogenizer, over a period of 10 minutes. After complete addition, the homogenizer was run for an additional 20 minutes. Finally, 0.1 gram of sodium bicarbonate was added to the mixture and blended with the homogenizer for 10 minutes.

A sample of the above aqueous emulsion was examined with the Fisher optical microscope as described in Comparative Example 1. The discontinuous "oil-phase" particles were observed to be of uniform size and approximately 250 nanometers in diameter. Samples of this emulsion were periodically inspected microscopically over the succeeding 3 months and were found to retain their particle size.

### EXAMPLE 2

An aqueous emulsion of liquid stearoxytrimethylsilane was prepared by the following method. Twelve (12) grams of water were mixed with 1.0 gram (1.330 mmole) of SilCareTM 140M40 Trisiloxane PEG-10 copolymer forming a clear solution (1). Forty grams of stearoxytrimethylsilane were mixed with 2.8 grams (0.272 mmole) of SilCareTM 140M30 Caprylyl bis(PEG/PPG-20/20) dimethicone copolymer until a hazy mixture was formed (II). Over a period of 10 minutes, mixture (II) was slowly added to solution (1) using a Tissue-Tearor homogenizer running in the range of 4000-8000 rpm. After complete addition, the homogenizer was run for an additional 20 minutes. To this mixture was added 45.1 grams of deionized water, using the homogenizer, over a period of 10 minutes. After complete addition, the homogenizer was run for an additional 20 minutes. Finally, 0.1 gram of sodium bicarbonate was added to the mixture and blended with the homogenizer for 10 minutes.

A sample of the above aqueous emulsion was examined with the Fisher optical microscope described in Comparative Example 1. The discontinuous "oil-phase" particles were observed to be of uniform size and approximately 250 nanometers in diameter. Samples of this emulsion were periodically inspected microscopically over the succeeding 3 months and were found to retain their particle size.

### EXAMPLE 3

An aqueous emulsion of liquid stearoxytrimethylsilane was prepared by the following method. Twelve (12) grams of water were mixed with 1.0 gram (1.330 mmole) of SilCareTM 140M40 Trisiloxane PEG-10 copolymer forming a clear solution (I). Forty grams of stearoxytrimethylsilane were mixed with 1.4 grams (0.136 mmole) of SilCareTM 140M30 Caprylyl bis(PEG/PPG-20/20) dimethicone copolymer until a hazy mixture was formed (11). Over a period of 10 minutes, mixture (II) was slowly added to solution (1) using a Tissue-Tearor homogenizer running in the range of 4000-8000 rpm. After complete addition, the homogenizer was run for an additional 20 minutes. To this mixture was added 45.6 grams of deionized water, using the homogenizer, over a period of 10 minutes. After complete addition, the homogenizer was run for an additional 20 minutes. Finally, 0.1 gram of sodium bicarbonate was added to the mixture and blended with the homogenizer for 10 minutes.

A sample of the above aqueous emulsion was examined with the Fisher optical microscope described in Comparative Example 1. The discontinuous "oil-phase" particles were observed to be of uniform size and approximately 250 nanometers in diameter. Samples of this emulsion were periodically inspected microscopically over the succeeding 3 months to retain their particle size.

### EXAMPLE 4

An aqueous emulsion of liquid stearoxytrimethylsilane in soybean oil was prepared by the following method. Twelve (12) grams of water were mixed with 0.5 grams (0.665 mmole) of SilCareTM 140M40 Trisiloxane PEG-10 copolymer forming a clear solution (1). A solution of 5.0 grams of stearoxytrimethylsilane in 35.0 Grams of soybean oil was mixed with 2.8 grams (0.272 mmole) of SilCareTM 140M30 Caprylyl bis(PEG/PPG-20/20) dimethicone copolymer forming a hazy mixture (11). Over a period of 10 minutes, mixture (11) was slowly added to solution (1) using a Tissue-Tearor homogenizer running in the range of 4000-8000 rpm. After complete addition, the homogenizer was run for an additional 20 minutes. To this mixture was added 44.7 grams of deionized water, using the homogenizer, over a period of 10 minutes. After complete addition, the homogenizer was run for an additional 20 minutes. Finally, 0.1 gram of sodium bicarbonate was added to the mixture and blended with the homogenizer for 10 minutes.

A sample of the above aqueous emulsion was examined with the Fisher optical microscope described in Comparative Example 1. The discontinuous "oil-phase" particles were observed to be of uniform size and approximately 250 nanometers in diameter. Samples of this emulsion were periodically inspected microscopically over the succeeding 3 months to retain their particle size.

The above-mentioned patents, patent applications and publications are hereby incorporated by reference.

Many variations of the invention will suggest themselves to those skilled in the art in light of the above-detailed description. All such obvious variations are within the full intended scope of the appended claims.

## Claims

1. An aqueous emulsion comprising:
a) a continuous phase comprising water;
b) a discontinuous phase comprising at least one liquid alkoxytrimethylsilane, wherein the discontinuous phase forms particles which are uniformly dispersed in the continuous phase; and
c) a dual emulsifier system consisting essentially of an organosilicone hydrophobic surfactant and an organosilicone hydrophilic surfactant.

2. The aqueous emulsion of claim 1, wherein the liquid alkoxytrimethylsilane is present in the emulsion in an amount ranging from 1 to 50 weight percent.

3. The aqueous emulsion of claim 2, wherein the liquid alkoxytrimethylsilane is present in the discontinuous phase of the emulsion in an amount ranging from 1 to 100 weight percent.

4. The aqueous emulsion of claims 1 to 3 wherein the alkoxytrimethylsilane is stearoxytrimethylsilane or retinoxytrimethylsilane.

5. The aqueous emulsion of claims 1 to 4, wherein the organosilicone hydrophobic surfactant is of an alkyl PEG/PPG-x/y dimethicone copolymer or an alkyl PEG/PPG-x/y methicone polymer, or an alkyl bis(PEG/PPG-x/y) dimethicone polymer, wherein alkyl is a saturated hydrocarbon substituent with from 6 to 18 carbons, PEG is polyoxyethylene, PPG is polyoxypropylene, and x and y may independently range from 1 to 20, and wherein said organosilicone hydrophobic surfactant is present in the emulsion at a concentration ranging from one to three molecules per 100 square Angstroms of surface area of the alkoxytrimethylsilane particles.

6. The aqueous emulsion of claims 1 to 5, wherein the organosilicone hydrophilic surfactant comprises a water soluble trisiloxane PEG-x copolymer, wherein the trisiloxane is (Me₃SiO)ZMeSi-, Me is methyl, PEG is polyoxyethylene, and x may range from 1 to 20, and wherein said organosilicone hydrophilic surfactant is present in the emulsion at a concentration of from two to six molecules per 100 square Angstroms of surface area of the organosilicone hydrophobic surfactant treated particles.

7. The aqueous emulsion of claims 1 to 6, wherein the organosilicone hydrophobic surfactant comprises a caprylyl bis-PEG/PPG-20/20 dimethicone copolymer or a trisiloxane PEG-10 copolymer.

8. The aqueous emulsion of claims 1 to 7, wherein the discontinuous phase is substantially comprised of particles having a diameter of less than about 500 nanometers.

9. The aqueous emulsion of claims 1 to 8, further comprising cosmetic components selected from the group consisting of a buffer, a biocide, a foaming agent, an anti-foaming agent, a fragrance, a colorant, a thickening agent and mixtures of any of the foregoing.

10. The aqueous emulsion of claims I to 9 wherein said discontinuous phase further comprises an inert organic diluent for the alkoxytrimethylsilane.

11. A cosmetic formulation for treatment of the skin or hair wherein the composition comprises an aqueous emulsion as defined in any of the preceding claims.

## Patentansprüche

1. Wässrige Emulsion, enthaltend:
a) eine Wasser enthaltende kontinuierliche Phase;
b) eine mindestens ein flüssiges Alkoxytrimethylsilan enthaltende diskontinuierliche Phase, die Teilchen bildet, die in der kontinuierlichen Phase einheitlich dispergiert sind; und
c) ein Dualemulgatorsystem, das im wesentlichen aus einem hydrophoben Organosilikon-Tensid und einem hydrophilen Organosilikon-Tensid besteht.

2. Wässrige Emulsion nach Anspruch 1, **dadurch gekennzeichnet, dass** das flüssige Alkoxytrimethylsilan in der Emulsion in einer Menge im Bereich von 1 bis 50 Gewichtsprozent vorliegt.

3. Wässrige Emulsion nach Anspruch 2, **dadurch gekennzeichnet, dass** das flüssige Alkoxytrimethylsilan in der diskontinuierlichen Phase der Emulsion in einer Menge im Bereich von 1 bis 100 Gewichtsprozent vorliegt.

4. Wässrige Emulsion nach den Ansprüchen 1 bis 3, **dadurch gekennzeichnet, dass** es sich bei dem Alkoxytrimethylsilan um Stearoxytrimethylsilan oder Retinoxytrimethylsilan handelt.

5. Wässrige Emulsion nach den Ansprüchen 1 bis 4, **dadurch gekennzeichnet, dass** es sich bei dem hydrophoben Organosilikon-Tensid um ein Alkyl-PEG/PPG-x/y-Dimethicon-Copolymer oder ein Alkyl-PEG/PPG-x/y-Methicon-Polymer oder ein Alkylbis(PEG/PPG-x/y)-Dimethicon-Polymer, worin Alkyl für einen gesättigten Kohlenwasserstoffsubstituenten mit 6 bis 18 Kohlenstoffatomen steht, PEG für Polyoxyethylen steht, PPG für Polyoxypropylen steht und x und y unabhängig voneinander im Bereich von 1 bis 20 liegen können, handelt und das hydrophobe Organosilikon-Tensid in der Emulsion in einer Konzentration im Bereich von einem bis drei Molekülen pro 100 Quadrat-Angström Oberfläche der Alkoxytrimethylsilan-Teilchen vorliegt.

6. Wässrige Emulsion nach den Ansprüchen 1 bis 5, **dadurch gekennzeichnet, dass** das hydrophile Organosilikon-Tensid ein wasserlösliches Trisiloxan-PEG-x-Copolymer, worin es sich bei dem Trisiloxan um (Me₃SiO)ZMeSi- handelt, Me für Methyl steht, PEG für Polyoxyethylen steht und x im Bereich von 1 bis 20 liegen kann, umfasst und in der Emulsion in einer Konzentration im Bereich von zwei bis sechs Molekülen pro 100 Quadrat-Angström Oberfläche der mit dem hydrophoben Organosilikon-Tensid behandelten Teilchen vorliegt.

7. Wässrige Emulsion nach den Ansprüchen 1 bis 6, **dadurch gekennzeichnet, dass** das hydrophobe Organosilikon-Tensid ein Caprylylbis-PEG/PPG-20/20-Dimethicon-Copolymer oder ein Trisiloxan-PEG-10-Copolymer umfasst.

8. Wässrige Emulsion nach den Ansprüchen 1 bis 7, **dadurch gekennzeichnet, dass** die diskontinuierliche Phase weitgehend aus Teilchen mit einem Durchmesser von weniger als etwa 500 Nanometer besteht.

9. Wässrige Emulsion nach den Ansprüchen 1 bis 8, ferner enthaltend kosmetische Komponenten aus der Gruppe bestehend aus einem Puffer, einem Biozid, einem Schäummittel, einem Antischaummittel, einem Riechstoff, einem Farbmittel, einem Verdickungsmittel und beliebigen Mischungen davon.

10. Wässrige Emulsion nach den Ansprüchen 1 bis 9, **dadurch gekennzeichnet, dass** die diskontinuierliche Phase ferner ein inertes organisches Verdünnungsmittel für das Alkoxytrimethylsilan enthält.

11. Kosmetikformulierung zur Behandlung der Haut oder der Haare, **dadurch gekennzeichnet, dass** die Zusammensetzung eine wässrige Emulsion gemäss einem der vorhergehenden Ansprüche umfasst.

## Revendications

1. Emulsion aqueuse comprenant :
a) une phase continue comprenant de l'eau ;
b) une phase discontinue comprenant au moins un alcoxytriméthylsilane liquide, la phase discontinue formant des particules qui sont dispersées uniformément dans la phase continue ; et
c) un système émulsifiant double constitué essentiellement d'un tensioactif hydrophobe de type organosilicone et d'un tensioactif hydrophile de type organosilicone.

2. Emulsion aqueuse selon la revendication 1, dans laquelle l'alcoxytriméthylsilane liquide est présent dans l'émulsion en quantité allant de 1 à 50 pour cent en poids.

3. Emulsion aqueuse selon la revendication 2, dans laquelle l'alcoxytriméthylsilane liquide est présent dans la phase discontinue de l'émulsion en quantité allant de 1 à 100 pour cent en poids.

4. Emulsion aqueuse selon les revendications 1 à 3, dans laquelle l'alcoxytriméthylsilane est le stéaroxytriméthylsilane ou le rétinoxytriméthylsilane.

5. Emulsion aqueuse selon les revendications 1 à 4, dans laquelle le tensioactif hydrophobe de type organosilicone est fait d'un copolymère alkyl-PEG/PPG-x/y diméthicone ou d'un copolymère alkyl-PEG/PPG-x/y méthicone, ou d'un copolymère alkyl-bis(PEG/PPG-x/y) diméthicone, dans laquelle le groupe alkyle est un substituant hydrocarboné saturé comportant de 6 à 18 atomes de carbone, PEG est un polyoxyéthylène, PPG est un polyoxypropylène, et x et y peuvent avoir indépendamment des valeurs de 1 à 20, et dans laquelle ledit tensioactif hydrophobe de type organosilicone est présent dans l'émulsion à une concentration allant d'une à trois molécules par unité de surface des particules d'alcoxytriméthylsilane de 100 angströms au carré.

6. Emulsion aqueuse selon les revendications 1 à 5, dans laquelle le tensioactif hydrophile de type organosilicone comprend un copolymère trisiloxane PEG-x soluble dans l'eau, dans laquelle le trisiloxane est (Me₃SiO)ZMeSi-, Me est un radical méthyle, PEG est un polyoxyéthylène, et x peut s'échelonner de 1 à 20, et dans laquelle ledit tensioactif hydrophile de type organosilicone est présent dans l'émulsion à une concentration de deux à six molécules par unité de surface de 100 angströms au carré des particules traitées avec le tensioactif hydrophobe de type organosilicone.

7. Emulsion aqueuse selon les revendications 1 à 6, dans laquelle le tensioactif hydrophobe de type organosilicone comprend un copolymère caprylyl-bis-PEG/PPG-20/20 diméthicone ou un copolymère trisiloxane PEG-10.

8. Emulsion aqueuse selon les revendications 1 à 7, dans laquelle la phase discontinue est essentiellement composée de particules ayant un diamètre inférieur à environ 500 nanomètres.

9. Emulsion aqueuse selon les revendications 1 à 8, comprenant en outre des constituants cosmétiques choisis dans le groupe constitué par un tampon, un biocide, un agent moussant, un agent antimousse, un parfum, un colorant, un agent épaississant, et des mélanges quelconques de ceux-ci.

10. Emulsion aqueuse selon les revendications 1 à 9 dans laquelle ladite phase discontinue comprend en outre un diluant organique inerte pour l'alcoxytriméthylsilane.

11. Formulation cosmétique pour le traitement de la peau ou des cheveux dans laquelle la composition comprend une émulsion aqueuse telle que définie dans l'une quelconque des revendications précédentes.
